(19) Europäisches Patentamt
European Patent Office
Office européen des brevets

(11) EP 2 801 361 A1

(12) # EUROPEAN PATENT APPLICATION
published in accordance with Art. 153(4) EPC

(43) Date of publication:
**12.11.2014 Bulletin 2014/46**

(21) Application number: **13733769.7**

(22) Date of filing: **04.01.2013**

(51) Int Cl.:
***A61K 31/4704*** $^{(2006.01)}$ ***A61P 9/00*** $^{(2006.01)}$
***C07D 215/22*** $^{(2006.01)}$

(86) International application number:
**PCT/JP2013/000001**

(87) International publication number:
**WO 2013/103148 (11.07.2013 Gazette 2013/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2012 JP 2012000706**

(71) Applicant: **Kowa Company Ltd.**
**Naka-ku**
**Nagoya-shi**
**Aichi 460-8625 (JP)**

(72) Inventors:
- **YOSHIDA, Hideo**
**Higashimurayama-shi**
**Tokyo 189-0022 (JP)**
- **ITOH, Shinsuke**
**Higashimurayama-shi**
**Tokyo 189-0022 (JP)**

(74) Representative: **Beckmann, Claus**
**Kraus & Weisert**
**Patentanwälte PartGmbB**
**Thomas-Wimmer-Ring 15**
**80539 München (DE)**

(54) # COLLATERAL BLOOD CIRCULATION DEVELOPMENT PROMOTER

(57) Provided is compound for improving blood flow at sites of impaired circulation in the extremities, through promoting development of collateral blood circulation without angiogenesis; also provided is a therapeutic agent for sensitivity to cold or cold sensation, in which the active ingredient is a compound for improving blood flow at sites of impaired circulation in the extremities. A collateral blood flow development promoter, in which the active ingredient is (-)-6-[3-[3-cyclopropyl-3-[(1R,2R)-2-hydroxycyclohexyl]ureido]propoxy]-2(1H)-quinolinone or a salt thereof, or a solvate thereof.



Printed by Jouve, 75001 PARIS (FR)

## Description

Technical field

The present invention relates to an agent for improving skeletal muscle blood flow in the extremities, wherein the active ingredient is (-)-6-[3-[3-cyclopropyl-3-[(1R,2R)-2-hydroxycyclohexyl]ureido]propoxy]-2(1H)-quinolinone, a salt thereof, or a solvate thereof, more specifically relates to a collateral blood circulation development promoter for skeletal muscles in the extremities.

Background Art

With the increase of lifestyle-related diseases due to the westernization of dietary habit, the morbidity of Peripheral Arterial Disease (PAD) has been increasing. Main disorders of PAD include Arteriosclerosis Obliterans (ASO) caused by arteriosclerosis, Thromboangiitis Obliterans (TAO, otherwise known as Buerger's disease) caused by vasculitis. ASO, which accounts for most chronic arterial occlusive diseases, is a condition that peripheral arteries in the extremities are chronically stenosed and occluded due to arteriosclerosis resulting in circulatory impairments, and represents ischemic clinical symptoms (cold sensation, intermittent claudication, feeling of numbness, and rest pain). The pathogenesis and development of PAD are mainly related to disorders of vascular endothelial cells, and deeply associated with the abnormal balance of various physiologically active substances. Furthermore, when PAD is progressed to be serious, it leads to critical limb ischemia (CLI), and develops trophic ulcer and gangrene if left untreated without proceeding revascularization procedure over the time, eventually resulting in lower-extremity amputation. The amputation of lower-extremity lowers Quality of Life (QOL) to a remarkable extent, and also adversely affects prognosis.

As for the clinical symptoms of ASO, most frequent manifestation is intermittent claudication, and about 1/3 of patients with ASO has typical claudication. Patients with intermittent claudication have normal blood flows at rest, however, the increase in muscle blood flow is limited during exercise. Therefore, the unbalance between oxygen supply and metabolism in muscles associated with claudication occurs and contributes to gait disturbance. It is said to be a close relation between an exercise (gait) capability and lower-extremity blood flow. Therefore, clinically, measurement of ankle-brachial index (ABI), lower-extremity blood flow, and blood oxygen saturation levels in muscles, and the like before and after exercise on a treadmill are used to diagnose clinical conditions and to confirm therapeutic efficacies (refer to Non-Patent Document 1, Non-Patent Document 2, Non-Patent Document 3).

The aim of the treatment of ASO through medication is to systemically improve arteriosclerosis in other organs and its risk factors, and to prevent cardiovascular events as well as to ameliorate ischemic clinical symptoms and to inhibit progression of ASO. In particular, on the subject of intermittent claudication that has the greatest number of patients, the improvements of exercise capability and QOL are extremely important because activities of daily life and QOL have become significantly impaired due to the deterioration of walking ability in patients with intermittent claudication. Therefore, for the treatment of PAD, exercise therapy, drug therapy, and surgical operation for revascularization at occlusion sites of vessels and the like have been carried out in addition to smoking abstinence, controlling risk factors such as diabetes mellitus, dyslipidemia, hypertension, hyperhomocysteinemia and the like. Moreover, for the appropriate treatment of claudication, improving hemodynamics in the lower-extremity and reducing the risk of lethal or non-lethal cardiovascular events occurring secondary to claudication are thought to be ideal (refer to Non-Patent Document 1).

There have been a lot of reports that platelet activation is involved in the development and progression of arteriosclerosis and thrombus formation following plaque rupture (refer to Non-Patent Document 4, and Non-Patent Document 5). However, there are no previous reports that anti-platelet agents such as aspirin and P2Y12 inhibitors have therapeutically beneficial effects on the treatment of intermittent claudication. According to Non-Patent Document 1, cilostazol that is a Phosphodiesterase 3 (PDE3) inhibitor and naftidrofuryl that is a 5-HT2 antagonist are recommended as the first-line pharmacotherapy agent to treat intermittent claudication. However, cilostazol is the only agent since the manufacturing and distribution of naftidrofuryl has been already discontinued.

Although surgical treatment for critical limb ischemia has been remarkably progressed, cases that surgical treatment is difficult to perform and those to which surgical treatment is not applicable most likely result in lower-extremity amputation. Therefore, there is much attention to therapeutic angiogenesis as a novel treatment method for such severe cases. Therapeutic angiogenesis is a method of promoting the development of collateral blood circulation by inducing angiogenesis using vascular growth factors and its genes, or bone marrow and peripheral blood cells, and improving ischemic condition. Vascular growth factors such as VEGF, HGF and fibroblast growth factor (FGF), and the like have potent vascular endothelial cell growth activities, and they are induced by stimulus such as ischemia and the like, and promote a novel capillary network. It is thought that angiogenesis by VEGF and etc. is associated with the increase of NO generation by the activation of eNOS via PI3K/Akt and EPC mobilization into a peripheral blood stream. Moreover, the activation of eNOS is also induced by the activation of cAMP/PKA and the stabilization of mRNA. Statin, a lipid-lowering drug, is proposed to activate eNOS, and the improving effect of statin on ischemia via angiogenesis in a rabbit

lower-extremity ischemic model is also reported. Moreover, in an in vitro experiment using human aortic endothelial cells (HAEC), cilostazol phosphorylates eNOS via cAMP/PKA and PI3K/Akt and promotes tubulogenesis of endothelial cells by increasing NO production. Thus, it is suggested that these effects could relate to the promotion of angiogenesis (refer to Non-Patent Document 6). Moreover, it has been reported that vascular density in lower-extremity muscles elevates in a lower-extremity ischemic mouse by a long-term administration of cilostazol (refer to Non-Patent Document 7). Therefore, cilostazol could possibly have an angiogenic activity, or the inhibitory activity of decreasing vascular density by ischemia.

On the other hand, it has been known that there are vessels that normally have no blood flow in a peripheral circulatory system, and those vessels develop a collateral blood circulation that could be called as the third blood vessel, when the blood flow from a peripheral artery to a vein is occluded (Non-Patent Document 8). As described above, the improvement of the blood flow by promoting the development of collateral blood circulation, which is different from angiogenesis, is thought to be an effective modality for the treatment of PAD. However, while the possibility that cilostazol has an angiogenic activity has been pointed out, the activity of promoting the development of collateral blood circulation without based on angiogenesis has not yet known.

The sensitivity to cold and cold sensation, in other words "poor circulation", is a state that the blood is not circulated to capillary blood vessels due to arteriosclerosis and etc., or that blood circulation is deteriorated due to the contraction of capillary blood vessels affected by temperature without recovering to the original condition. It is particularly a state that has the cold feeling of limbs. The deterioration of the function of the autonomic nerve, low blood pressure, anemia, and the like also impair the blood flow and cause the sensitivity to cold. In any of these cases, energy does not circulate to capillary blood vessels due to poor blood flow and creates "the condition of getting cold". Symptoms such as headache due to poor circulation, stiffness in the shoulder, chalk mark, diarrhea and abdominal pain, heavy stomach feeling due to autonomic nerve disorders, menstrual irregularity, and the like also appear.

Meanwhile, a compound represented by the following formula (1), wherein

[Chemical Formula 1]

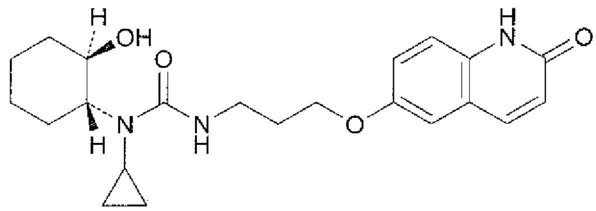

(1)

(-)-6-[3-[3-cyclopropyl-3-[(1R,2R)-2-hydroxycyclohexyl]ureido]propoxy]-2(1H)-quinolinone (hereinafter sometimes referred to as "Compound 1") has an antiplatelet activity, an antithrombogenic activity and an inhibitory effect of PDE3. As its indications, ischemic disorders including brain disorders such as cerebral atherosclerosis, cerebral infarction, transient ischemic attacks, reversible ischemic neurological deficit, and the like; cardiac diseases such as cardiac infarction, angina pectoris, and the like; chronic arterial occlusive diseases such as Buerger's disease, arteriosclerosis obliterans, intermittent claudication, and the like; complications of diabetes mellitus such as diabetic neuropathy, diabetic cutaneous ulcer, diabetic nephropathy, and the like; prevention of restenosis after procedures such as percutaneous transluminal coronary angioplasty (PTCA), directional coronary atherectomy (DCA) and stent intervention; the prevention of reocclusion of artificial organs such as artificial blood vessels, kidneys, and the like after transplantation procedures; or the prevention of thrombogenesis and embolism that could occur after operations and during dialysis and the like with extracorporeal circulation are known (Non-Patent Document 9, and Patent Document 1). However, it has not been known that the Compound 1 has a promoting activity of the development of collateral blood circulation in the extremities and has beneficial effect to sensitivity to cold and cold sensation.

Citation List

Patent Document

Patent Document 1: WO97/012869

Non-Patent Documents

Non-Patent Document 1: Diagnosis·treatment guideline II of lower-extremity arteriosclerosis obliterans (TASC II) 2007, Japanese College of Angiology Edit.
Non-Patent Document 2: J. Jpn. Coll. Angiol. 45, 312-316 (2005)
Non-Patent Document 3: The Japanese Journal of Thrombosis and Hemostasis, 19(1), 45-47 (2008)
Non-Patent Document 4: Atherosclerosis., 187, 221-237 (2006)
Non-Patent Document 5: J Intern Med., 263, 517-527 (2008)
Non-Patent Document 6: Atherosclerosis., 189, 350-357 (2006)
Non-Patent Document 7: Eur J Vasc Endovasc Surg. 2011 Aug 16. [Epub ahead of print]
Non-Patent Document 8: Annals of the Royal College of Surgeons of England, 161-176 (1953)
Non-Patent Document 9: Biochem. Biophys. Research. Commun., 353(4), 1111-1114 (2007)

Summary of the Invention

Problems to be Solved by the Invention

The present invention provides a compound of improving blood flow at sites of impaired circulation in the extremities through promoting the development of collateral blood circulation without angiogenesis. In addition, the present invention provides a therapeutic agent for the treatment of sensitivity to cold or cold sensation, wherein the active ingredient is a compound that improves blood flow at sites of impaired circulation in the extremities.

Means for Solving the Problems

The inventors have strenuously studied to solve the above-mentioned problems, and have found that the compound represented by said formula (1) improves blood flow at sites of impaired circulation in the extremities through promoting the development of collateral blood circulation without angiogenesis, and have achieved the present invention.
More specifically, the present invention provides an agent for improving blood flow in the extremities, wherein the active ingredient is (-)-6-[3-[3-cyclopropyl-3-[(1R,2R)-2-hydroxycyclohexyl]ureido]propoxy]-2(1H)-quinolinone (Compound 1) or a salt thereof, or a solvate thereof. More preferably, the present invention provides a collateral blood circulation development promoter, wherein the active ingredient is the Compound 1 or a salt thereof, or a solvate thereof, preferably a collateral blood circulation development promoter for improving blood flow at sites of impaired circulation in the extremities, more preferably a collateral blood circulation development promoter for improving blood flow at sites of impaired circulation in the lower-extremity. Further preferably, the present invention provides a collateral blood circulation development promoter without based on angiogenesis, wherein the active ingredient is the Compound 1 or a salt thereof, or a solvate thereof.
In addition, the present invention provides a prophylactic and/or therapeutic agent for sensitivity to cold, wherein the active ingredient is (-)-6- [3-[3-cyclopropyl-3-[(1R,2R)-2-hydroxycyclohexyl]ureido]propoxy]-2(1H)-quinolinone or a salt thereof, or solvates thereof.

Effects of the Invention

According to the present invention, (-)-6-[3-[3-cyclopropyl-3-[(1R,2R)-2-hydroxycyclohexyl]ureido]propoxy]-2(1H)-quinolinone or a salt thereof, or a solvate thereof, as described in Examples below, has the improving effect on hindlimb skeletal muscle blood flow, in particular, has a promoting activity of the development of collateral blood circulation in hindlimb skeletal muscle without relying on angiogenesis. It is useful for preventing or treating symptoms associated with peripheral arterial disease, for example, sensitivity to cold and cold feeling.
The treatment method for peripheral arterial disease (PAD) includes exercise therapy such as gait training (refer to Non-Patent Document 3), and it is thought that gait training lead to the development of collateral blood circulation and then result in improvement of blood flow. The Compound 1 in the present invention promotes the development of collateral blood circulation by drug administration, so that provides a novel treatment method for peripheral arterial disease (PAD).

Brief Description of Drawings

FIG. 1 illustrates the result examining the effect of the Compound 1 on the proliferative activity of human aortic endothelial cells (HAEC). Values are represented as mean ± standard error (n = 3).
FIG. 2 is a photograph showing the result examining the effect of the Compound 1 on tube formation of normal

human umbilical vein endothelial cells (HUVEC).

FIG. 3 illustrates the result examining the effect of the Compound 1 on tube formation of HUVEC. Values are represented as mean (n = 2).

FIG. 4 illustrates the effectiveness of the rat femoral artery occlusion model prepared in this experiment with respect to blood flow volume in the gastrocnemius muscle. The blood flow volume in the gastrocnemius muscle shown in FIG. 4 was evaluated on Day 28 after the operation; it shows blood flow deficit in the gastrocnemius muscle before exercise (the left side in FIG. 4) and after exercise (the right side in FIG. 4) in the rat femoral artery occlusion model in comparison to sham-operated controls. Values are represented as mean ± standard error (n = 7), wherein *** indicates a significant difference at the $P < 0.001$ level.

FIG. 5 illustrates the effectiveness of the rat femoral artery occlusion model prepared in this experiment with respect to blood flow volume in the anterior tibialis muscle. The blood flow volume in the anterior tibialis muscle shown in FIG. 5 was evaluated on Day 28 after the operation, it shows blood flow deficit in the anterior tibialis muscle before exercise (the left side of FIG. 5) and after exercise (the right side of FIG. 5) in the rat femoral artery occlusion model in comparison to the sham-operated controls. Values are represented as mean ± standard error (n = 7), wherein *** indicates a significant difference at the $P < 0.001$ level.

FIG. 6 illustrates the effects of repeated oral administration of the Compound 1 twice daily for 27 days to rat femoral artery occlusion model prepared in this experiment on increase of blood flow volume in the gastrocnemius muscle before exercise (the left side of FIG. 6) and after exercise (the right side of FIG. 6). Values are represent as mean ± standard error (n = 20), wherein *** indicates a significant difference at the $P < 0.001$ level.

FIG. 7 illustrates the effects of repeated oral administration of the Compound 1 twice daily for 27 days to rat femoral artery occlusion model prepared in this experiment on increase of blood flow volume in the anterior tibialis muscle before exercise (the left side of FIG. 7) and after exercise s(the right side of FIG. 7). Values are represented as mean ± standard error (n = 20), wherein *** indicates a significant difference at the $P < 0.001$ level.

FIG. 8 illustrates the effect of the Compound 1 on intramuscular arteries in the rat femoral artery occlusion model. The Compound 1 was orally administered twice daily for 27 days in the rat femoral artery occlusion model prepared in this experiment, and the luminal area of intramusclar artery in the gastrocnemius muscle was measured. Values are represented as mean ± standard error (n = 16), wherein * indicates a significant difference at the $P < 0.05$ level, and *** indicates a significant difference at the $P < 0.001$ level.

FIG. 9 illustrates vasodilation of the rat femoral artery by the Compound 1. Values are represented as mean ± standard error (n = 3).

## Modes for Carrying Out the Invention

The Compound 1 in the present invention is publicly known, and it can be produced according to the method described in WO97/012869, or a method that is equivalent to this.

Moreover, a salt of the Compound 1 or a solvate thereof can be used in the present invention. The salt and solvate of the Compound 1 can be produced by a conventional method.

As the salt of the Compound 1 in the present invention, a base addition salt can be recited, but is not particularly restricted as far as it is a pharmaceutical acceptable salt. Such a salt includes, for example, base addition salt with alkali metals such as potassium, sodium and the like; and alkali earth metals such as magnesium, calcium, and the like.

As the solvate of the Compound 1 in the present invention, a hydrate, an alcohol solvate (for example, ethanol solvate), and the like can be recited.

In the present description, a lower-extremity circulatory disorder animal model means an animal that is induced circulatory impairment in lower-extremities by ligation, excision of lower-extremity vessels, occlusion of peripheral blood vessels by the intravascular injection of a inducing agent for platelet aggregation or a drug, occlusion of peripheral blood vessels by electro stimulation to the vessels, occlusion of peripheral blood vessels by laser radiation to the vessels, stenosis of blood vessels by cuff, and the like. Specifically, for example, a laurate-induced peripheral circulation disorder model, a lower-extremity arterial ligation peripheral circulation disorder model, an ergotamine-induced peripheral circulation disorder model, an ergotamine + epinephrine-induced peripheral circulation disorder model, and the like are recited. More preferable model includes the laurate-induced peripheral circulation disorder model, and the lower-extremity arterial ligation peripheral circulation disorder model.

As for exercise in the present description, exercises on a treadmill for animals or a ROTA-ROD, nerve stimulation under anesthesia, and the like can be recited, exercises on the treadmill for animals and the ROTA-ROD are more preferred, exercises on the treadmill for animals are particularly preferred. The treadmill for animals provides continuous exercise stress to a tested animal that keeps them run on a belt-driven belt with inclined angle. Exercise on the treadmill in the present invention is given at a belt speed of 5 to 40 m/min, preferably at 15 to 25 m/min, with a belt inclined angle of 5 to 40 degrees, preferably 10 to 20 degrees, and the duration of exercise is 30 sec to 30 min, preferably 1 to 10 min.

Colored microspheres used for the measurement of hindlimb blood flow in the present description comprises

98% of polystyrene and 2% of divinylbenzene with an average diameter of 15.5 μm. They are labeled with a special dye that has a peak absorbance at particular wavelength, and the number of microspheres can be converted by the measurement of the absorbance of its stained solution with a spectrophotometer. When the microspheres are administered into the left ventricular through the carotid artery of the animal, blood flow volume in tissues can be calculated by the extraction of microspheres accumulated in the tissues since microspheres are distributed in the tissues in proportion to each blood flow volume in the tissues.

The collateral blood circulation development promoter in the present invention promotes the development of collateral blood circulation at sites of circulatory impairments, preferably it is for improving the blood flow at sites of impaired circulation in the extremities, more preferably it is for improving blood flow at sites of impaired circulation in the lower-extremity.

In addition, the collateral blood circulation development promoter in the present invention activates an alternative route that is preexisted to support blood flow to tissues and reflux to the heart for the case that the circulation of arteries and veins are occluded, and promotes development of said alternative route not by de novo angiogenesis.

The collateral blood circulation development promoter in the present invention, in particular, improves blood flow at sites of impaired circulation in the extremities, and has beneficial effect of improving, treating and/or preventing symptoms of peripheral arterial disease (PAD) such as arteriosclerosis obliterans (ASO) and thromboangiitis obliterans (TAO).

A pharmaceutical agent in the present invention can be prepared alone or using pharmaceutically acceptable carriers such as dissolving agent, excipient, bonding agent, and diluent as dosage forms such as tablets, capsules, granules, powdered formulation, lotion, ointment, injections, and suppository, and the like. These drug formulations can be produced by a publicly known method. For example, dissolving agents such as tragacanth gum, Gum arabic, sucrose ester, lecithin, olive oil, soybean oil, PEG 400, and the like; excipients such as starch, mannitol, lactose, and the like; bonding agents such as sodium carboxymethyl cellulose, hydroxypropyl cellulose, and the like; disintegrating agents such as crystalline cellulose, calcium carboxymethyl cellulose, and the like; lubricants such as talc, magnesium stearate, and the like; flow improvers such as light anhydrous silicic acid, and the like; and diluents such as corn starch, and the like are arbitrarily combined and prescribed to produce formulations for oral administration.

A pharmaceutical agent in the present invention is administered orally or parenterally. Dose of the pharmaceutical agent in the present invention varies depending on body weight, age, sex, and condition of patients, but for adult it is usually administered at a dose of 0.01 to 1000 mg, preferably 25 to 100 mg per day, wherein the daily dosage is preferably divided into 1 to 3 times.

## Examples

Hereinafter, the present invention is specifically explained by Examples, however, the present invention is not limited to these Examples.

### Example 1

Study of the effect of Compound 1 on vascular endothelial cells

(1) Drugs and cells used in the experiment

The Compound 1 was produced according to the method described in WO97/012869 and dissolved in dimethyl sulfoxide (solvent). As for cells used in the experiment, human aortic endothelial cells (HAEC (Lonza Group Ltd.)) and normal human umbilical vein endothelial cells (HUVEC; American Type Culture Collection) were used.

(2) Study of the effect of the Compound 1 on the proliferative activity of HAEC

HAEC were cultured using an endothelial cell growth medium (EGM-2 bullet kit; Lonza) containing 10% fetal bovine serum (FBS). The proliferative activity of HAEC was assessed by a WST-8 assay using a Cell Counting Kit (Dojindo Laboratories). In a 96-well plate, HAEC were seeded at a concentration of 5000 cells per well, and incubated for 24 hours. Subsequently, the medium in each well was exchanged to 100 μL of fresh culture medium containing various concentrations of the Compound 1 or solvent alone. After the 48 hours of subsequent incubation, 10 μL of WST-8 reagent was added to each well. The plate was incubated another 4 hours, and then the absorbance at 450 nm was measured with an absorbance plate reader.

(3) Study of the effect of the Compound 1 on HUVEC tube formation

HUVEC were cultured using an endothelial cell growth medium (Endothelial Cell Growth Medium: Cell Applications) containing 10% FBS. In a 96-well plate coated with Matrigel matrix, HUVEC were seeded at a concentration of $1.5 \times 10^4$ cells per well, and incubated in a 200 μL of culture medium containing various concentrations of the Compound 1 or solvent alone for 18 hours. After that, total tube length in a field (Total tube length) was calculated by image analysis using a light microscope (E-400; Nikon Corp.).

(4) Statistical analysis

Values were expressed as mean ± standard error, or mean. All the statistical analyses were carried out using SAS9.1.3 (SAS Institute Japan Ltd.). In all the analyses, $P < 0.05$ indicates a statistical significance.

(5) Results

1. Study of the effect of the Compound 1 on the proliferative activity of HAEC

FIG. 1 shows diagrammatically the effect of the Compound 1 on the proliferative activity of HAEC. The vertical axis in FIG. 1 represents the number of cells in a unit at a wavelength of 450 nm (Arbitrary number/OD450); the horizontal axis indicates control containing solvent alone, 1 μM and 3 μM of the Compound 1 from the left of the horizontal axis. From this result, there was no significant difference, and no effect of the Compound 1 on the proliferative activity of HAEC was observed.

2. Study of the effect of the Compound 1 on HUVEC tube formation

The results of the effect of the Compound 1 on HUVEC tube formation are shown in photographs in FIG. 2 and shown in a graph of Total tube length in FIG. 3. The photographs of FIG. 2 show the results obtained when cells were incubated with solvent alone as control, 0.01 μM of the Compound 1, 0.1 μM of the Compound 1, 1 μM of the Compound 1, 10 μM of the Compound 1, and 100 μM of the Compound 1, respectively. From these photos, no angiogenesis could be confirmed. Moreover, when vessels are newly generated, vasculature construction in early stage such as the proliferation of endothelial cells and tube formation by fusing endothelial cells each other is important. The result of measurement of tube formation is shown in the graph of FIG. 3. The vertical axis of FIG. 3 indicates the ratio of Total tube length with respect to the control (%); the horizontal axis indicates each concentration (μM) of the Compound 1. From this result, there is no significant difference with respect to the control, and the effect of the Compound 1 on HUVEC tube formation was not observed.

3. Angiogenic activity of the Compound 1

These results demonstrated that the Compound 1 does not have an angiogenic activity via direct action to endothelial cells.

Example 2

Effect of the Compound 1 on blood flow of lower limb gastrocnemius muscle in a rat femoral artery ligation model

(1) Drugs and animals used in the experiment

The Compound 1 was produced according to the method described in WO97/012869 and was used by dissolving in dimethyl sulfoxide or suspending in 1% (W/W) of hydroxypropyl methyl cellulose aqueous solution. As for animals, 7 to 9 week-old Sprague-Dawley (Slc:SD) male rats were used. As for anesthesia for operation, pentobarbital·sodium was intraperitoneally administered at a dose of 50 mg/kg.

(2) A rat hindlimb ischemia model by ligation of femoral artery

Bilateral femoral arteries were exposed and the arteries were ligated using a suture under anesthesia. For histological analysis, the Compound 1 or base alone was orally administered twice daily for 27 days starting on the day of operation. On Day 28, the left gastrocnemius muscle was extirpated and frozen in a Tissue-Tek O.C.T. compound (Sakura Finetech Japan Co., Ltd.). Tissue sections at a thickness of 10 μm were prepared by slicing the frozen tissue

sample with a freezing microtome, and dried in an air and fixed with acetone. The fixed tissue sections were treated with 5% goat serum to block non-specific binding of antibodies, and then were reacted with a rabbit antibody against α-smooth muscle actin (α-SMA) as the first reaction, were reacted with biotin labeled goat anti-rabbit IgG (Vector Laboratories Inc.) as the second reaction, and were reacted with horseradish peroxidase (HRP) labeled streptavidin (KPL, Kirkegaard & Perry Laboratories, Inc.) as the third reaction to form immunocomplexes. This immunocomplexes were visualized by the enzyme reaction of HRP that was used diaminobenzidine as a substrate. The positive staining with α-SMA was identified by an examination using a light microscope, and the luminal area of intramuscular artery was quantified by image analysis (Win ROOF; Mitani Co., Ltd.). The blood flow volume on Day 28 in the hindlimb muscle in this model was determined by measuring at two points, in other words before and after exercise, using Dye-Trak VII+ microspheres (Triton Technology, Inc.).

In particular detail, initially a pentobarbital·sodium solution was intraperitoneally administered to the rat at a dose of 50 mg/kg to anesthetize. Next, the rat was laid at an abdominal position on a thermal insulation mat for body temperature and the body temperature was maintained at 37°C using a heat pad (BWT-100, Bio Research Center Co., Ltd.). Then, the hip part of the rat was incised, and the left sciatic nerve was exfoliated and exposed, and two silk ligatures were hung over the nerve for ligation performed later. The rat was placed on a dorsal position, a catheter filled with saline containing proper dose of heparin was inserted to the left brachial artery and detained followed by connection to a blood pressure transducer. After that, a catheter filled with saline containing proper dose of heparin was inserted to the right common carotid artery, and the distal end of the catheter was placed in the left ventricular, and connected with a blood pressure transducer (DX-360, KOHDEN Engineering Co., Ltd.) to measure intracardiac pressure. Wave profile of blood pressure that is characteristic to the left ventricular was detected to confirm whether the catheter has reached to the left ventricular. The temporal pulse pressure and average blood pressure were measured with a sphygmomanometer via the blood pressure transducer. The measurement of heart rate was conducted by measuring blood pressure-pulse triggering a temporal heart rate tachometer. Furthermore, these data were recorded on thermal paper. After confirming that blood pressure and heart rate of the rat were stabilized, the periphery site of the brachial artery indwelling catheter was connected to a 1 mL-disposable syringe containing proper amount of heparin, and the syringe was attached to a diffusion pump. Next, a left ventricular indwelling catheter was filled with a yellow microspheres solution (0.2 mL, 600,000 particles), and the distal end of the catheter was connected to a 5 mL-disposable syringe filled with saline, and the syringe was loaded to an injection pump. From the left ventricular indwelling catheter, the yellow microspheres solution followed by 0.5 mL of saline were injected at the speed of 0.3 mL/min over a period of 140 seconds in total. One minute after exercise, a reference blood sample was retrieved and Persimmon-colored microspheres solution (0.2 mL, 600,000 particles) was injected in the same manner as mentioned above.

Next, the rats were euthanized with an overdose of the pentobarbital·sodium solution, and then the left gastrocnemius muscle was retrieved. Immediately after that, the wet weight of the muscle was measured and the muscle was transferred into a 15 mL polypropylene tube. The retrieved reference blood sample and gastrocnemius muscle were stored at 4°C until recovery test of microspheres is conducted. Saline containing 2% Tween 80 was added to a 15 mL tube containing the reference blood sample, and the mixture was diluted in measuring up to 9 mL in total, and added 3 mL of 16 mol/L KOH. Twelve mL of 4 mol/L KOH was added to a 15 mL tube containing the gastrocnemius muscle. Next, 9990 vesicles of blue microspheres were added to both sample tubes containing the blood and gastrocnemius muscle to measure extraction efficiency of microspheres. Both sample tubes were incubated at 60°C for 3 hours or more and subjected to alkaline lysis. Degraded tissue solution was filtered in vacuo using a filter and an aspiration pump, and microspheres were retrieved on a polytetrafluoroethylene filter (10 μm pore size, 25 mm diameter). The filter was then washed with 10 mL of purified water that had been prewarmed at 60°C three times, further washed with 10 mL of ethanol once, dried, and transferred to a 1.5 mL polypropylene tube. To this tube was added 310 μL of acidic Cellosolve acetate and the coloring dye was extracted from the microspheres. This 1.5 mL tube was then centrifuged under the condition of 22°C at 2000G for 5 minutes, and 200 μL of the supernatant was transferred to a 96-well microplate. The absorbance was measured with an Absorbance Plate Reader at 440 nm (maximum absorption wavelength of yellow microsphere), 545 nm (maximum absorption wavelength of persimmon-color microsphere), and 672 nm (maximum absorption wavelength of blue microsphere). The absorbance values were entered to Excel macro for the analysis of blood flow volume (A Dye-Trak matrix inversion macro for Microsoft Excel, Triton Technology, Inc.), the number of microspheres contained in each blood sample and the gastrocnemius muscle sample was computed. The blood flow volume in the gastrocnemius muscle Qg (mL/min/g) was computed by formula:

$$Qg = (Ng \times Qr)/(Nr \times Wg)$$

wherein, Ng represents the number of microspheres contained in the gastrocnemius muscle sample, Qr represents the yield of the reference blood sample (mL/min), Nr represents the number of microspheres contained in the reference

blood sample, and Wg represents the wet weight value (g) of the gastrocnemius muscle.

(3) Study of vasodilation in the femoral artery

The femoral artery vessel (3 mm in width) excised from the rat was placed to a Magnus tube filled with a Krebs-Henseleit solution maintained at 37°C, to which a mixed gas of 95% $O_2$ and 5% $CO_2$ was continuously injected by reference to the method by Abiru et al. (Abiru T, et al., Eur J Pharmacol. 1995; 281:9-15.). The isometric tension of the vessel was measured with a tension transducer, and the result was recorded with a thermal printer. The vessel was adjusted to a resting tension of 0.75 g. The vessel was contracted by 60 mmol/L (rats) of potassium chloride, and then the Compound 1 or a base was continuously injected to the Magnus tube. At the end of each experiment, 100 mmol/L of papaverine was added in order to determine the maximum relaxation.

(4) Pharmacokinetics study of the Compound 1 in the rat

The Compound 1 was orally administered in a single dose to the rats (n = 3) under non-fasting condition to determine the concentration-time profile of the serum collected from the inferior vena cava. The concentration of the Compound 1 in the serum was detected with a high-performance liquid chromatography system attached with a fluorescence detector. The maximum plasma concentration (Cmax) and time to the maximum plasma concentration (Tmax) were determined from the measurement of the drug concentration in the serum, half-life ($T_{1/2}$), and AUC. The time curve was computed by a least squares method and a trapezoidal method, respectively.

(5) Statistical analysis

Values were expressed as mean ± standard error. All the statistical analysis are conducted using SAS9.1.3 (SAS Institute Japan Ltd.). In all the analyses, $P < 0.05$ indicates a significant difference.

(6) Result 1. Increase of hindlimb muscle blood flow by the Compound 1

The result of the change in blood flow in the gastrocnemius muscle (hindlimb muscle) is shown in the graph of FIG. 4. The vertical axis of FIG. 4 indicates the blood flow (mL/min/g), the left side of the horizontal axis indicates the blood flow before exercise, the right side indicates one after exercise, respective shams indicate sham-operated groups, and ligations indicate ligation groups. As a result, the blood flow in the gastrocnemius muscle (hindlimb muscle) in the rat hindlimb ischemia model prepared by femoral artery ligation was significantly reduced compared to the sham-operated group after conducting exercise on Day 28 (in comparison to 0.515 ± 0.067 mL/min/g in the ligation group, 1.492 ± 0.145 mL/min/g was observed in the sham-operated group showing significant difference at the $P < 0.001$ level. n = 7).

In addition, the change in the blood flow in anterior tibialis muscle (hindlimb muscle) was shown in the graph of FIG. 5. The vertical axis of FIG. 5 indicates the blood flow (mL/min/g), at the left side of the horizontal axis indicates blood flow before exercise, at the right side indicates one after exercise, respective shams indicate sham-operated groups, and ligations indicate ligation groups. From this result, the blood flow in anterior tibialis muscle (hindlimb muscle) in the rat hindlimb ischemia model prepared by femoral artery ligation was also significantly reduced compared to the sham-operated group after exercise on Day 28 (in comparison to 0.516 ± 0.098 mL/min/g in the ligation group, 1.687 ± 0.163 mL/mim/g was observed in the sham-operated group showing a significant difference at the $P < 0.001$ level. n = 7).

By contrast the result observed after exercise, there was no significant difference between both groups regarding the blood flow before exercise (at rest) (refer to FIG. 4 and FIG. 5). This is consistent with the result reported in Brevetti LS, et al., J Surg Res. 2001; 98:21-61. In other words, in the hindlimb ischemia model, the increase of the blood flow after exercise was defected in the skeletal muscle blood flow, therefore, it is suggested that this animal model reflects the pathology of human intermittent claudication.

Next, the effect of the Compound 1 (30 mg/kg) was assessed using this model when it was orally administered twice daily for 27 days at repeated dose. It was observed that the Compound 1 increased the blood flow in the gastrocnemius muscle and anterior tibialis muscle after exercise on Day 28 compared to the control.

The result of the change in the blood flow in the gastrocnemius muscle (hindlimb muscle) was shown in the graph of FIG. 6. The vertical axis of FIG. 6 indicates the blood flow (mL/min/g), at the left part of the horizontal axis indicates that before exercise, the right part indicates that after exercise, the left sides of each part indicate control groups administered with solvent only, the right sides of each part indicate the Compound 1 (30 mg/kg) administration groups. From this result, while the blood flow in the gastrocnemius muscle in the control group showed 0.561 ± 0.034 mL/min/g, that in the Compound 1 administration group showed 0.818 ± 0.058 mL/min/g, and significant difference was observed at the $P < 0.001$ level (n = 20).

Moreover, a change in the blood flow of the anterior tibialis muscle was shown in the graph of FIG. 7. The

vertical axis of FIG. 7 indicates the blood flow (mL/min/g), the left part of the horizontal axis indicates that before exercise, the right part indicates that after exercise, the left sides of each part indicate the control groups administered with solvent only, the right sides of each part indicate the Compound 1 (30 mg/kg) administration groups. From this result, the blood flow in anterior tibialis muscle showed 0.697 ± 0.039 mL/min/g in the control group, that in the Compound 1 administration group showed 0.992 ± 0.060 mL/min/g, and significant difference was observed at the $P < 0.001$ level (n = 20).

However, in any cases, there was no change in the hindlimb muscle blood flow before exercise (refer to FIG. 6 and FIG. 7).

2. Enlargement of hindlimb intramuscular arteries by the Compound 1

The result of measuring the luminal area of the main artery in the gastrocnemius muscle in the same rat hindlimb ischemia model is shown in the graph of FIG. 8. The vertical axis of FIG. 8 indicates the luminal area ($\mu m^2$) of the main artery in the gastrocnemius muscle. The horizontal axis of FIG. 8 indicates a sham-operated group, a control group administered with solvent only that underwent ligation, and a Compound 1 (30 mg/kg) administration group that underwent ligation from the left of the horizontal axis, respectively. From this result, the control group underwent ligation showed 3315 ± 416 $\mu m^2$ showing a significant decrease compared to 14642 ± 1062 $\mu m^2$ in the sham-operated control group ($P < 0.001$, n = 16). This stenosis in the intramuscular arteries was significantly suppressed when the Compound 1 (30 mg/kg) was orally administered twice daily for 27 days at repeated dose (7100 ± 824 $\mu m^2$, $P < 0.05$, n = 16).

3. Vasodilation of the femoral arteries by the Compound 1

The result of the vasodilation of the femoral arteries by the Compound 1 is shown in the graph of FIG. 9. The vertical axis of FIG. 9 indicates the ratio of vasodilation (%), and the horizontal axis indicates drug concentration (μmol/L). White open triangles in FIG. 9 indicate the control group administered with solvent only, and black filled circles indicate the Compound 1 administration group. This result showed that the Compound 1 (0.1 μmol/L to 30 μmol/L) had a dose-dependent vasodilating effect on the ring specimen of the excised rat femoral artery that was contracted with KCl in the Magnus tube in vitro.

4. Pharmacokinetics study of the Compound 1 in the rat

Pharmacokinetics study of the Compound 1 in the rat was carried out. This result is shown in the following Table 1. From this result, the blood concentration of the Compound 1, which was equivalent to the concentration that can exert enough vasodilating effect observed in FIG. 9 by single oral administration of the Compound 1 at a dose of 30 mg/kg to a rat, was obtained (Cmax = 4.73 ± 0.28 μmol/L) (refer to Table 1).

These results suggested that the improving effect of the Compound 1 on lower-extremity circulatory disorder observed in FIG. 6, FIG. 7, and FIG. 8 would be related to the blood flow increase through the vasodilating effect of the Compound 1.

[Table 1]

| Dose (mg/kg) | Cmax (μmol/L) | AUC (0-t) (μmol× hr/L) | AUC (0-inf) (μmol× hr/L) | Tmax (hr) | $T_{1/2}$ (hr) |
|---|---|---|---|---|---|
| 3 | 0.30 ± 0.06 | 0.83 | 1.33 | 1.0 | 2.64 |
| 10 | 0.83 ± 0.14 | 2.38 | 2.55 | 1.0 | 2.00 |
| 30 | 4.73 ± 0.28 | 14.09 | 14.79 | 2.0 | 1.61 |
| 100 | 9.76 ± 1.44 | 47.11 | 48.24 | 1.0 | 2.19 |

These results showed that the Compound 1 improved the hindlimb skeletal muscle blood flow in the rat peripheral vascular disease model. This effect is thought to appear through a mechanism that is different from an angiogenesis promoting activity, which was reported by a result when cilostazol was administered. Moreover, other antiplatelet agents such as aspirin and P2Y12 inhibitors have not been shown to have a beneficial effect to the treatment of intermittent claudication.

Industrial Applicability

The Compound 1 in the present invention has an action of improving blood flow at sites of impaired circulation in the extremities via the promotion of development of collateral blood circulation, and is beneficial to prevent and treat

various symptoms including intermittent claudication such as, for example, sensitivity to cold or cold sensation, which are attributed to peripheral arterial disease and the like, and the Compound 1 has the potential of industrial applicability.

## Claims

1. A collateral blood circulation development promoter, wherein the active ingredient is (-)-6-[3-[3-cyclopropyl-3-[(1R,2R)-2-hydroxycyclohexyl]ureido]propoxy]-2(1H)-quinolinone or a salt thereof, or a solvate thereof.

2. The collateral blood circulation development promoter according to Claim 1, wherein the promotion of the development of collateral blood circulation is not based on angiogenesis.

3. The collateral blood circulation development promoter according to Claim 1 or 2, wherein the collateral blood circulation development promoter is for improving blood flow at sites of impaired circulation in the extremities.

4. A prophylactic and/or therapeutic agent for sensitivity of cold, wherein the active ingredient is (-)-6-[3-[3-cyclopropyl-3-[(1R,2R)-2-hydroxycyclohexyl]ureido]propoxy]-2(1H)-quinolinone or a salt thereof, or a solvate thereof.

FIG. 1

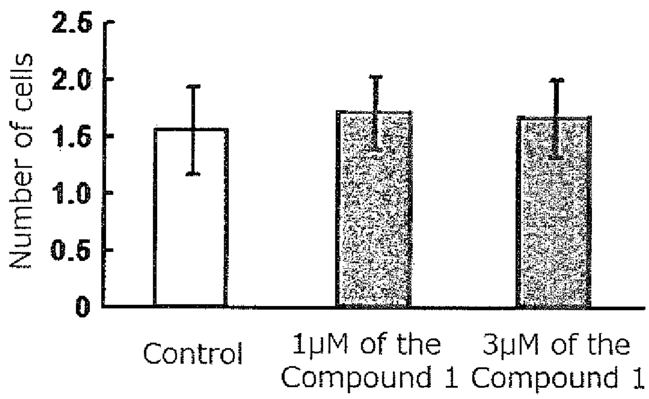
2.5
2.0
1.5
1.0
0.5
0
Number of cells
Control
1μM of the Compound 1
3μM of the Compound 1

FIG. 2

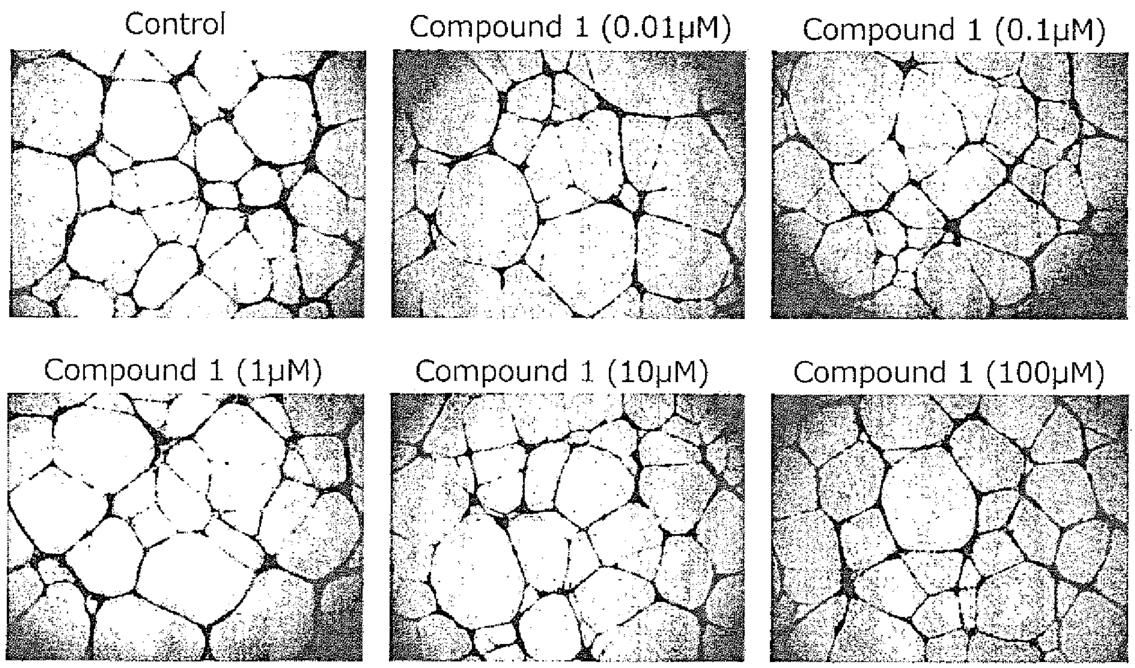
Control
Compound 1 (0.01μM)
Compound 1 (0.1μM)
Compound 1 (1μM)
Compound 1 (10μM)
Compound 1 (100μM)

FIG. 3

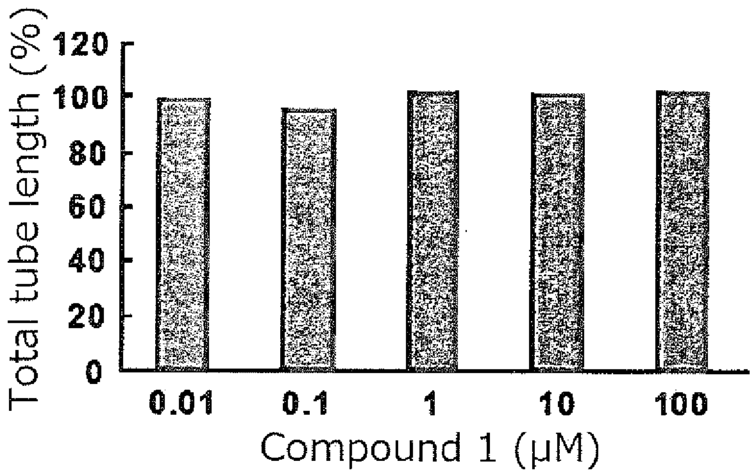
Total tube length (%)
120
100
80
60
40
20
0
0.01
0.1
1
10
100
Compound 1 (μM)

FIG. 4

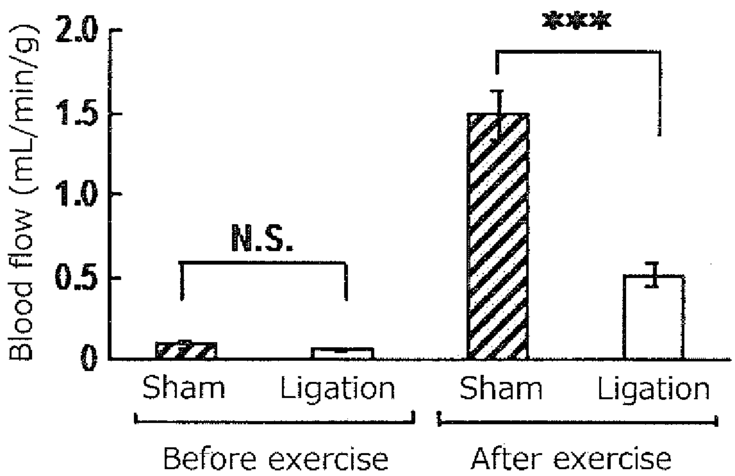
Blood flow (mL/min/g)
2.0
1.5
1.0
0.5
0
N.S.
***
Sham
Ligation
Sham
Ligation
Before exercise
After exercise

FIG. 5

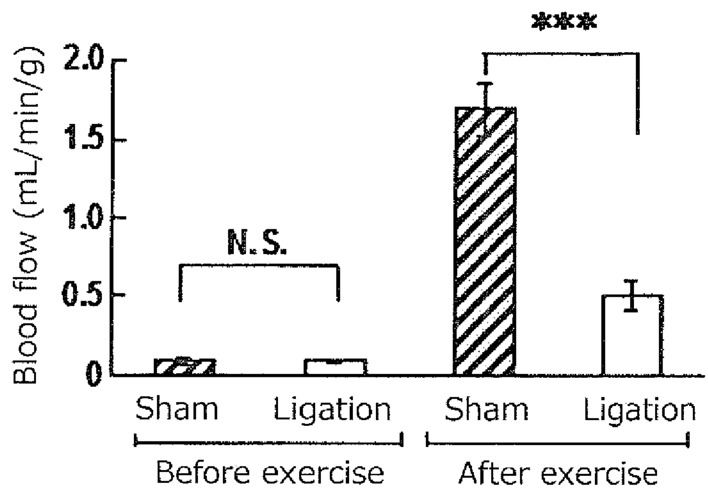
***
2.0
1.5
1.0
0.5
0
Blood flow (mL/min/g)
N.S.
Sham
Ligation
Sham
Ligation
Before exercise
After exercise

FIG. 6

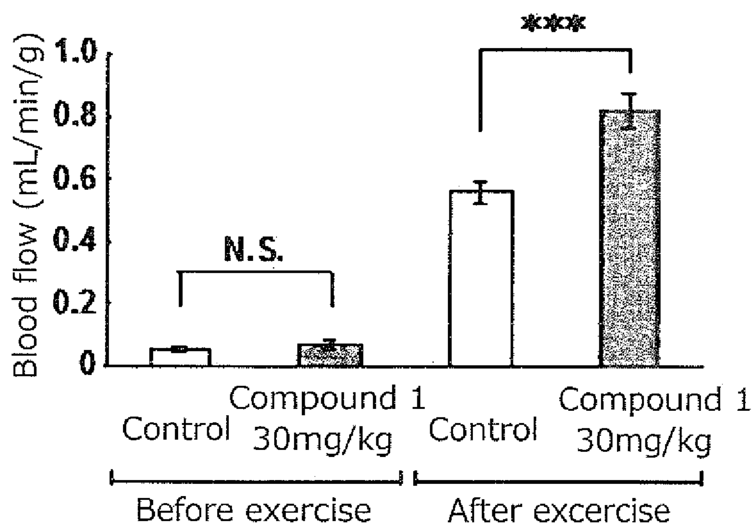
***
1.0
0.8
0.6
0.4
0.2
0
Blood flow (mL/min/g)
N.S.
Control
Compound 1 30mg/kg
Control
Compound 1 30mg/kg
Before exercise
After excercise

FIG. 7

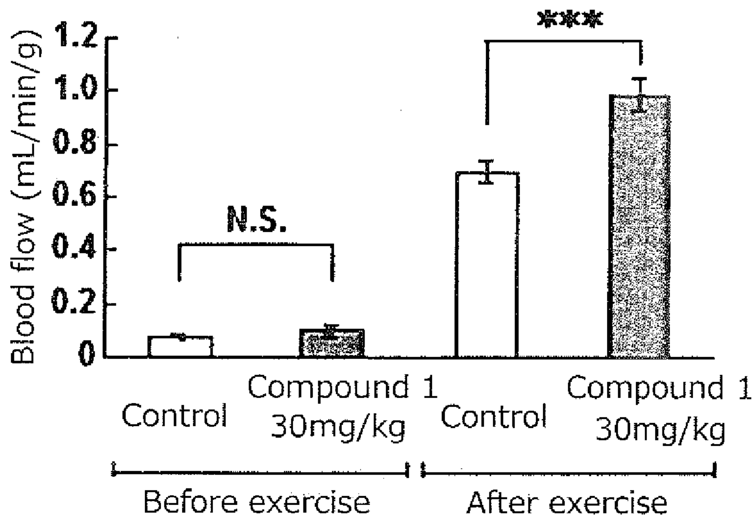
***
N.S.
Blood flow (mL/min/g)
1.2
1.0
0.8
0.6
0.4
0.2
0
Control
Compound 1
30mg/kg
Control
Compound 1
30mg/kg
Before exercise
After exercise

FIG. 8

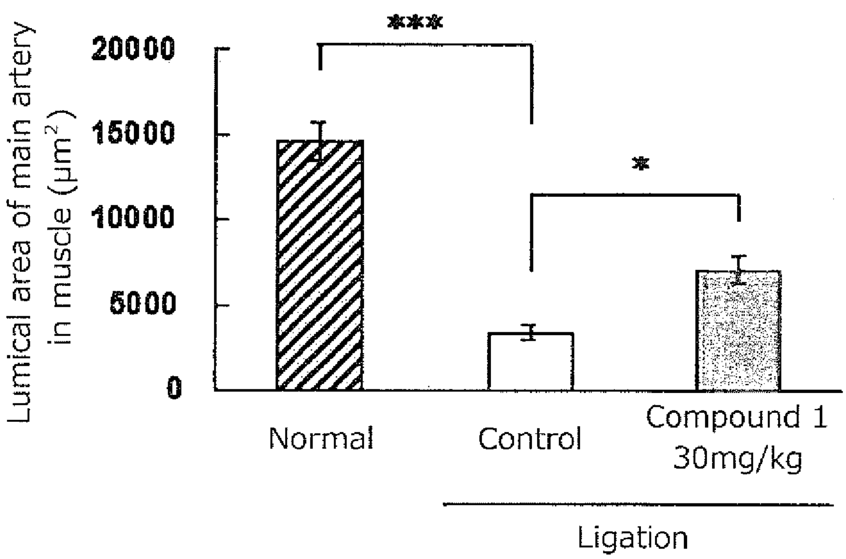
***
*
Lumical area of main artery
in muscle (μm²)
20000
15000
10000
5000
0
Normal
Control
Compound 1
30mg/kg
Ligation

FIG. 9

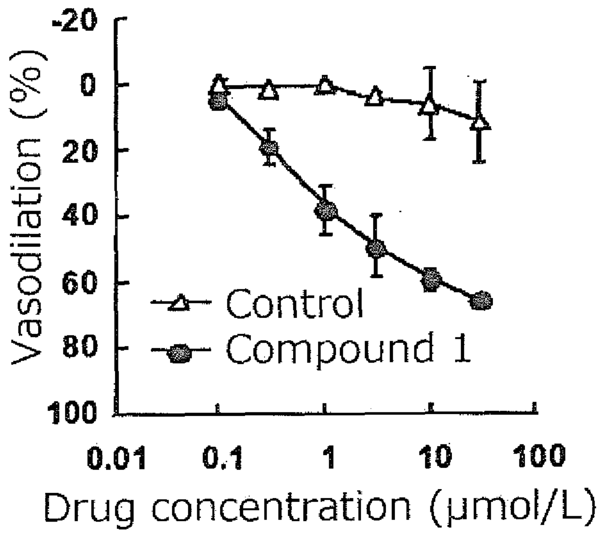
-20
0
20
40
60
80
100
Vasodilation (%)
Control
Compound 1
0.01
0.1
1
10
100
Drug concentration (μmol/L)

## INTERNATIONAL SEARCH REPORT

International application No.
PCT/JP2013/000001

A. CLASSIFICATION OF SUBJECT MATTER
*A61K31/4704*(2006.01)i, *A61P9/00*(2006.01)i, *C07D215/22*(2006.01)n

According to International Patent Classification (IPC) or to both national classification and IPC

B. FIELDS SEARCHED

Minimum documentation searched (classification system followed by classification symbols)
A61K31/4704, A61P9/00, C07D215/22

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus/REGISTRY/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | JP 09-157258 A (OTSUKA PHARM CO., LTD.), 17 June 1997 (17.06.1997), entire text; particularly, claims; paragraphs [0027] to [0029] & WO 97/12869 A1 & EP 796248 A1 & JP 2964029 B2 & US 6143763 A & EP 796248 B1 | 1-3 |
| X<br>Y | LEWIS,R.J. et al., Application of adaptive design and decision making to a phase II trial of a phosphodiesterase inhibitor for the treatment of intermittent claudication, TRIALS, 2011, vol.12, no.134, pp.1-9, entire text, particularly, Abstruct, page 2, left column, lines 33 to 36 | 1-3<br>1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 January, 2013 (28.01.13) | 05 February, 2013 (05.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2013/000001

C (Continuation). DOCUMENTS CONSIDERED TO BE RELEVANT

| Category* | Citation of document, with indication, where appropriate, of the relevant passages | Relevant to claim No. |
|---|---|---|
| Y | Hiroyuki KOYAMA, "Sokufuku Kekkoro no Hattatsu to Kino - Kekkan Shinsei Ryoho Kaihatsu no Shiten kara", Angiology Frontier, 2004, vol.3, no.1, pages 78 to 80, entire text, particularly, page 78, left column, lines 6 to 14, page 80 | 1-4 |
| Y | Shigeru TERAMOTO et al., "Cilostazol and treatment of occlusive arteriosclerosis and Buerger disease", Therapeutic Research, 1990, vol.11, no.2, pages 40 to 44, entire text, particularly, page 40, right column, lines 12 to 26 | 1-4 |
| P,X | YOSHIDA,H. et al., A phosphodiesterase 3 inhibitor, K-134, improves hindlimb skeletal muscle circulation in rat models of peripheral arterial disease, Atherosclerosis, 2012 (Available online 2012.01.05), vol.221, pp.84-90, entire text, particularly, Abstruct | 1-4 |
| P,X | SASAKI Y. et al., K-134, a phosphodiesterase 3 inhibitor, improves gait disturbance and hindlimb blood flow impairment in rat peripheral artery disease models, European Journal of Pharmacology, 2012(Available online 2012.05.31), vol.689, pp.132-138, entire text, particularly, Abstruct | 1-4 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 97012869 A


### Non-patent literature cited in the description


- Diagnosis·treatment guideline II of lower-extremity arteriosclerosis obliterans (TASC II). *Japanese College of Angiology Edit,* 2007
- *J. Jpn. Coll. Angiol.,* 2005, vol. 45, 312-316
- *The Japanese Journal of Thrombosis and Hemostasis,* 2008, vol. 19 (1), 45-47
- *Atherosclerosis,* 2006, vol. 187, 221-237
- *J Intern Med.,* 2008, vol. 263, 517-527
- *Atherosclerosis,* 2006, vol. 189, 350-357
- *Eur J Vasc Endovasc Surg.,* 16 August 2011
- Annals of the Royal College of Surgeons of England. 1953, 161-176
- *Biochem. Biophys. Research. Commun.,* 2007, vol. 353 (4), 1111-1114
- **ABIRU T et al.** *Eur J Pharmacol.,* 1995, vol. 281, 9-15
- **BREVETTI LS et al.** *J Surg Res.,* 2001, vol. 98, 21-61